(19) Europäisches Patentamt
European Patent Office
Office européen des brevets

(11) EP 4 324 815 A1

(12) EUROPÄISCHE PATENTANMELDUNG

(43) Veröffentlichungstag:
**21.02.2024 Patentblatt 2024/08**

(21) Anmeldenummer: **22190843.7**

(22) Anmeldetag: **17.08.2022**

(51) Internationale Patentklassifikation (IPC):
***C07C 29/151*** ^(2006.01)^ ***C07C 31/04*** ^(2006.01)^
***C25B 1/04*** ^(2021.01)^ ***C01B 3/36*** ^(2006.01)^

(52) Gemeinsame Patentklassifikation (CPC):
(C-Sets verfügbar)
**C07C 29/1518; C01B 3/00; C01B 3/36; C25B 1/04; C25B 15/08** (Forts.)

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**KH MA MD TN**

(71) Anmelder: **L'AIR LIQUIDE, SOCIETE ANONYME POUR L'ETUDE ET L'EXPLOITATION DES PROCEDES GEORGES CLAUDE**
**75007 Paris (FR)**

(72) Erfinder:
- **Oelmann, Tobias**
**60439 Frankfurt am Main (DE)**
- **Schuhmann, Timm**
**64625 Bensheim (DE)**
- **Linicus, Matthias**
**60439 Frankfurt am Main (DE)**

(74) Vertreter: **Stang, Stefan**
**Air Liquide Forschung und Entwicklung GmbH**
**Gwinnerstraße 27-33**
**60388 Frankfurt am Main (DE)**

# (54) VERFAHREN UND ANLAGE ZUR HERSTELLUNG VON METHANOL UND SYNTHESEGAS

(57) Erfindungsgemäß wird ein Verfahren und eine Anlage zur Herstellung von kohlendioxidbasiertem Methanol und von Synthesegas vorgeschlagen, wobei das erzeugte Synthesegas prozessintern für die Methanolsynthese genutzt werden kann. In einem Kohlendioxid-Einsatzgasstrom als Verunreinigung enthaltene und unter den Bedingungen der Methanolsynthese inerte Kohlenwasserstoffe werden stofflich durch die Integration einer Reformierungseinheit, insbesondere einer POX-Einheit, verwertet. Dies erfolgt durch die Zuführung eines aus der Methanolsynthese-Schleife abgezweigten Spülgasstroms zu genannter Reformierungseinheit, in welcher die Kohlenwasserstoffe zu Synthesegas umgesetzt werden. In einer bevorzugten Ausführungsform wird der Spülgasstrom einer Wasserstoffrückgewinnungseinheit, insbesondere einer Membraneinheit, zugeführt. Der retentatseitig erzeugte, mit Kohlenwasserstoffen angereicherte Spülgasstrom wird anschließend der Reformierungseinheit zugeführt.

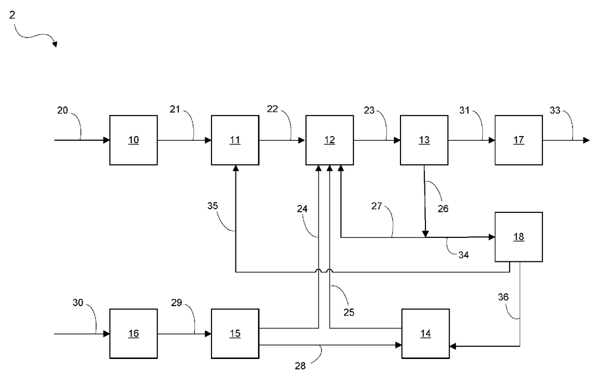


Fig. 2



Processed by Luminess, 75001 PARIS (FR)

(Forts. nächste Seite)

(52) Gemeinsame Patentklassifikation (CPC): (Forts.)

C-Sets
**C07C 29/1518, C07C 31/04**

## Beschreibung

### Gebiet der Erfindung

Die Erfindung betrifft ein Verfahren und eine Anlage zur Herstellung von Methanol aus kohlendioxidreichem Synthesegas und zur Herstellung von Synthesegas als Nebenprodukt der Methanolherstellung, wobei das als Nebenprodukt gebildete Synthesegas prozessintern für die Methanolherstellung genutzt werden kann.

### Stand der Technik

Methanol wird im großtechnischen Maßstab aus Synthesegas hergestellt. Synthesegas ist üblicherweise ein Gemisch aus vorwiegend Wasserstoff ($H_2$), Kohlenmonoxid (CO) sowie Kohlendioxid ($CO_2$). Kohlenmonoxid sowie Kohlendioxid werden dabei häufig unter dem Begriff "Kohlenoxide" oder "Kohlenstoffoxide" zusammengefasst. Dabei laufen an einem festen Methanolsynthese-Katalysator vornehmlich die beiden folgenden Gleichgewichtsreaktionen (1) und (2) nebeneinander ab.

$$(1) \qquad CO_2 + 3\,H_2 \rightleftharpoons CH_3OH + H_2O$$

$$(2) \qquad CO + 2\,H_2 \rightleftharpoons CH_3OH$$

Methanol kann auch aus Synthesegas hergestellt werden, welches arm an oder sogar frei von Kohlenmonoxid ist. Entsprechend kommt dann vorwiegend Reaktionsgleichung (1) bei der Methanolsynthese zur Geltung. Die Herstellung dieses sogenannten kohlendioxidbasierten Methanols spielt insbesondere bei Prozessen eine Rolle, welche ohne oder zumindest mit möglichst wenigen fossilen Energieträgern oder fossilen Einsatzstoffen realisiert werden sollen und möglichst wenige Treibhausgasemissionen erzeugen sollen. Ein Beispiel für einen solchen Prozess offenbart die WO 2016/034344 A1.

So kann kohlendioxidbasiertes Methanol beispielsweise auf der Basis von durch Verbrennung eines fossilen Einsatzstoffes erzeugtem Kohlendioxid und regenerativ erzeugtem Wasserstoff hergestellt werden. Der regenerativ erzeugte Wasserstoff wird vorzugsweise durch Elektrolyse von Wasser auf Basis regenerativ erzeugten Stroms hergestellt.

Das Kohlendioxid kann aus verschiedensten Quellen stammen, wie zum Beispiel aus Abgasen der Chemie-, Stahl- und Zementindustrie sowie aus Biogasen und Erdgasen. Das Kohlendioxid kann dabei durch Kohlenstoffabscheidung (engl. *carbon capture*) aus dem jeweiligen Gas aufkonzentriert werden. Bekannte und im industriellen Maßstab bereits verwendete Kohlenstoffabscheideverfahren sind physikalische und chemische Absorption in einem geeigneten Lösungsmittel, kryogene Partialkondensation und Trennung der Gase durch Membranen. Ein bekanntes physikalisches Abscheideverfahren ist die je nach Verfahrensführung mehr oder wenige selektive Absorption des Kohlendioxids in tiefkaltem Methanol unter erhöhtem Druck mit anschließender Desorption durch Druckabsenkung und gegebenenfalls Strippen mit geeigneten Strippgasen. Ein bekanntes chemisches Abscheideverfahren ist die Absorption des Kohlendioxids an ein Amin einer wässrigen Aminlösung, mit anschließender Desorption des Kohlendioxids durch Erhitzen.

Ein für die Methanolsynthese zu nutzender Kohlendioxidstrom kann verschiedene Verunreinigungen, insbesondere Kohlenwasserstoffe, enthalten. Die Kohlenwasserstoffe können dabei aus verschiedenen Komponenten zusammengesetzt sein, wie beispielsweise Methan und andere niedere Alkane ($<C_{10}$), niedere Olefine, aromatische Verbindungen, alicyclische Verbindungen und Furane.

Bevor ein Kohlendioxidstrom als Einsatzgasstrom für eine Methanolsynthese genutzt werden kann, kann die Aufreinigung des Kohlendioxidstroms erforderlich sein. Dabei können die Kohlenwasserstoffe durch geeignete Reinigungsverfahren entweder abgeschieden oder zersetzt werden. In beiden Fällen wird der in den Kohlenwasserstoffen gebundene Wasserstoff und Kohlenstoff stofflich anschließend nicht genutzt. Werden die Kohlenwasserstoffe nicht abgeschieden oder zersetzt, gelangen diese als (unter den Bedingungen der Methanolsynthese) inerte Komponenten zusammen mit dem Kohlendioxid und Wasserstoff in die Methanolsynthese. Da industrielle Prozesse zur Methanolsynthese immer eine Syntheseschleife mit Führung der Prozessgase im Kreislauf beinhalten, akkumulieren die Kohlenwasserstoffe in diesem Kreislauf und müssen letztlich mit dem sogenannten Spülstrom (engl. *purge*) aus dem Kreislauf ausgeschleust werden. Vom Spülstrom kann optional Wasserstoff als Wertgas abgetrennt werden, und das verbleibende Gas wird beispielsweise zur Unterfeuerung in einem anderen oder der Methanolsynthese vorgeschalteten Prozess genutzt. Somit wird auch in diesem Fall zumindest der in den Kohlenwasserstoffen gebundene Kohlenstoff stofflich nicht genutzt.

## Beschreibung der Erfindung

Eine allgemeine Aufgabe der vorliegenden Erfindung besteht darin ein Verfahren oder eine Anlage vorzuschlagen, welche(s) die Nachteile des Standes der Technik zumindest teilweise überwindet.

Eine Aufgabe der vorliegenden Erfindung besteht darin ein Verfahren oder eine Anlage vorzuschlagen, welche(s) die stoffliche Nutzung des in Kohlenwasserstoffen gebundenen Kohlenstoffs und Wasserstoffs bezüglich der Synthese von Methanol verbessert.

Eine Aufgabe der vorliegenden Erfindung besteht darin ein Verfahren oder eine Anlage vorzuschlagen, welche(s) die stoffliche Nutzung des in Kohlenwasserstoffen gebundenen Kohlenstoffs und Wasserstoffs bezüglich der kohlendioxidbasierten Synthese von Methanol verbessert.

Ein Beitrag zur mindestens teilweisen Erfüllung mindestens einer der obigen Aufgaben wird durch die unabhängigen Ansprüche geleistet. Die abhängigen Ansprüche stellen bevorzugte Ausführungsformen bereit, die zur mindestens teilweisen Erfüllung mindestens einer der Aufgaben beitragen. Bevorzugte Ausgestaltungen von Bestandteilen einer erfindungsgemäßen Kategorie sind, soweit zutreffend, ebenso bevorzugt für gleichnamige oder entsprechende Bestandteile einer jeweils anderen erfindungsgemäßen Kategorie. Die Ausdrücke "aufweisend", "umfassend" oder "beinhaltend" etc. schließen nicht aus, dass weitere Elemente, Inhaltsstoffe etc. enthalten sein können. Der unbestimmte Artikel "ein" schließt nicht aus, dass eine Mehrzahl vorhanden sein kann.

Die vorgenannten Aufgaben werden zumindest teilweise gelöst durch ein Verfahren zum Herstellen von Methanol und Synthesegas, umfassend die folgenden Verfahrensschritte:

(a) Bereitstellen eines kohlenwasserstoffhaltigen Kohlendioxidstroms;

(b) Bereitstellen eines elektrolytisch erzeugten Wasserstoffstroms;

(c) Zusammenführen der Ströme aus Schritt (a) und (b) zu einem kohlenwasserstoffhaltigen Synthesegasstrom;

(d) Umsetzen des kohlenwasserstoffhaltigen Synthesegasstroms und eines Kreislaufgasstroms in einem Methanolsynthese-Reaktor, wobei ein Rohmethanolstrom als Reaktionsprodukt und ein Restgasstrom erhalten werden, wobei der Restgasstrom nicht zu Methanol umgesetztes Synthesegas und Kohlenwasserstoffe enthält;

(e) Auftrennen des Restgasstroms in den Kreislaufgasstrom und einen Spülgasstrom;

(f) Umsetzen des Spülgasstroms unter Beteiligung von Sauerstoff als Oxidationsmittel in einem Reformierungsschritt zu einem Synthesegasstrom.

Erfindungsgemäß wird der in den Kohlenwasserstoffen gebundene Kohlenstoff und Wasserstoff stofflich genutzt, indem der kohlenwasserstoffhaltige Spülgasstrom in einem Reformierungsschritt unter Beteiligung von Sauerstoff zu einem Synthesegasstrom umgesetzt wird. Insbesondere werden die Kohlenwasserstoffe des Spülgasstroms in einem Reformierungsschritt unter Beteiligung von Sauerstoff zu einem Synthesegasstrom umgesetzt. Der Spülgasstrom enthält neben den Kohlenwasserstoffen auch nicht im Methanolsynthese-Reaktor umgesetztes Kohlendioxid und Wasserstoff. Der durch den Reformierungsschritt erzeugte Synthesegasstrom enthält Wasserstoff, Kohlenmonoxid und Kohlendioxid.

Der kohlenwasserstoffhaltige Synthesegasstrom gemäß Schritt (c) erzeugt weist Synthesegas auf. Ein Synthesegas ist ein Gasgemisch, welches zumindest ein Kohlenstoffoxid (Kohlenmonoxid oder Kohlendioxid) und Wasserstoff aufweist. Vorzugsweise weist das Synthesegas des kohlenwasserstoffhaltigen Synthesegasstroms gemäß Schritt (c) erzeugt Kohlendioxid und Wasserstoff auf. Der gemäß Schritt (f) erzeugte Synthesegasstrom weist Synthesegas auf. Vorzugsweise weist das Synthesegas des Synthesegasstroms gemäß Schritt (f) erzeugt Kohlenmonoxid, Kohlendioxid und Wasserstoff auf.

Unter einem Reformierungsschritt wird grundsätzlich die chemische Umsetzung von Kohlenwasserstoffen mit Sauerstoff und/oder Dampf zu einem Synthesegas verstanden. Gemäß dem Reformierungsschritt gemäß Schritt (f) werden die Kohlenwasserstoffe des Spülgasstroms zumindest unter Beteiligung von Sauerstoff zu Synthesegas umgesetzt, optional zusätzlich mit Dampf zu Synthesegas umgesetzt.

Der durch Reformierung der Kohlenwasserstoffe des Spülgasstroms erzeugte Synthesegasstrom kann neben dem kohlenwasserstoffhaltigen Synthesegasstrom für die Methanolsynthese genutzt werden.

Eine Ausführungsform des erfindungsgemäßen Verfahrens ist daher dadurch gekennzeichnet, dass der gemäß Schritt (f) erzeugte Synthesegasstrom zusätzlich zum kohlenwasserstoffhaltigen Synthesegasstrom im Methanolsynthese-Reaktor umgesetzt wird.

Der gemäß Schritt (f) erzeugte Synthesegasstrom weist Kohlenmonoxid auf. Da Kohlenmonoxid gegenüber Wasserstoff eine bessere Reaktivität bei der Methanolsynthese aufweist als Kohlendioxid, ergibt sich für die prozessinterne Nutzung des gemäß Schritt (f) erzeugten Synthesegasstroms ein entsprechend vorteilhafter Effekt.

Bei dem Reformierungsschritt handelt es sich vorzugsweise um eine partielle Oxidation. Eine bevorzugte Ausführungsform des erfindungsgemäßen Verfahrens ist daher dadurch gekennzeichnet, dass der Reformierungsschritt eine partielle Oxidation beinhaltet.

Die partielle Oxidation (POX) wird vorzugsweise in einem dafür konfigurierten Reaktor oder POX-Reaktor durchgeführt. POX-Reaktoren können klein dimensioniert werden und eignen sich daher auch für kleinere Stoffströme, was bei dem umzusetzenden Spülgasstrom regelmäßig der Fall ist.

Eine partielle Oxidation läuft für Methan als Kohlenwasserstoff zunächst nach folgender Reaktionsgleichung (3) ab.

$$(3)\ CH_4 + 1\tfrac{1}{2}\, O_2 \rightleftharpoons CO + 2\, H_2O$$

Die exotherme partielle Oxidation liefert Dampf und die erforderliche Wärmeenergie für eine nachfolgende endotherme Dampfreformierungsreaktion, welche nicht katalysiert nach Reaktionsgleichung (4) abläuft.

$$(4)\ CH_4 + H_2O \rightleftharpoons 3\, H_2 + CO$$

Dem POX-Reaktor kann zur Einstellung eines optimalen Kohlenstoff-Dampf-Verhältnisses zusätzlich Dampf zugeführt werden.

Bei der katalysierten Variante von Gleichung (4) spricht man von autothermer Reformierung, was von der "reinen" partiellen Oxidation in einem POX-Reaktor zu unterscheiden ist.

Die Dampfreformierungsreaktion wird von der Wassergas-Shift-Reaktion gemäß Reaktionsgleichung (5) begleitet, welche exotherm verläuft.

$$(5)\ CO + H_2O \rightleftharpoons CO_2 + H_2$$

Die Hauptelemente eines POX-Reaktors sind ein Brenner und eine Brennkammer, die sich in einem feuerfest ausgekleideten Druckmantel befinden. In einem POX-Reaktor findet die partielle Oxidation des kohlenwasserstoffhaltigen Einsatzstroms stets durch unterstöchiometrische Mengen Sauerstoff statt. Die Temperatur des Gasgemisches am Ausgang des Reaktors liegt typischerweise im Bereich von 1250 bis 1450 Grad Celsius. Der Prozessdruck liegt typischerweise bei 20 bis 100 bar. Erfindungsgemäß ist eine Aufreinigung des Kohlendioxidstroms in Bezug auf die im Kohlendioxidstrom enthaltenen Kohlenwasserstoffe nicht erforderlich, da diese im Rahmen von Schritt (f) stofflich genutzt werden. Erfindungsgemäß wird daher gemäß Schritt (a) ein kohlenwasserstoffhaltiger Kohlendioxidstrom bereitgestellt. Darüber hinaus wird gemäß Schritt (b) ein elektrolytisch erzeugter Wasserstoffstrom bereitgestellt. Beide Ströme werden zusammengeführt, wodurch ein kohlenwasserstoffhaltiger Synthesegasstrom gemäß (c) resultiert. Dieser Synthesegasstrom enthält zumindest Kohlendioxid und Wasserstoff und Kohlenwasserstoffe als Verunreinigung. Darüber hinaus kann dieser Synthesegasstrom auch Kohlenmonoxid enthalten.

Gemäß einem Beispiel liegt der Anteil von Kohlenwasserstoffen im kohlenwasserstoffhaltigen Kohlendioxidstrom bei 0,1 bis 10 %, oder 1 bis 10 %, oder bei 2 bis 8 %. Der Anteil an Kohlendioxid im kohlenwasserstoffhaltigen Kohlendioxidstrom kann stark variieren, je nachdem ob der Kohlendioxidstrom in einem vorhergehenden Prozessschritt, insbesondere in einer Kohlenstoffabscheideeinheit, aufkonzentriert wurde. Entsprechend kann der Anteil des Kohlendioxids im kohlenwasserstoffhaltigen Kohlendioxidstrom beispielsweise in einem Bereich von 50 Vol.-% bis 99,5 Vol.-% liegen. Vorzugsweise weist der kohlenwasserstoffhaltige Kohlendioxidstrom somit einen Kohlendioxidanteil von 50 Vol.-% bis 99,5 Vol.-% auf, oder einen Kohlendioxidanteil von 75 Vol.-% bis 99,5 Vol.-% auf, oder einen Kohlendioxidanteil von 90 Vol.-% bis 99,5 Vol.-% auf, oder einen Kohlendioxidanteil von 95 Vol.-% bis 99,5 Vol.-% auf.

Der gemäß Schritt (c) erzeugte kohlenwasserstoffhaltige Synthesegasstrom ist von dem durch Schritt (f) erzeugten Synthesegasstrom zu unterscheiden. Für diesen Zweck kann der gemäß Schritt (c) erzeugte kohlenwasserstoffhaltige Synthesegasstrom als "erster Synthesegasstrom" und der gemäß Schritt (f) erzeugte Synthesegasstrom als "zweiter Synthesegasstrom" bezeichnet werden.

Der kohlenwasserstoffhaltige Synthesegasstrom und ein Kreislaufgasstrom werden gemäß Schritt (d) in einem Methanolsynthese-Reaktor umgesetzt. Insbesondere werden die im kohlenwasserstoffhaltigen Synthesegasstrom und Kreislaufgasstrom enthaltenen Komponenten Wasserstoff und Kohlendioxid sowie optional Kohlenmonoxid zu Rohmethanol als erwünschtes Reaktionsprodukt umgesetzt.

Die Umsetzung im Methanolsynthese-Reaktor erfolgt bei Synthesedruck, beispielsweise einem Druck von 40 bar bis 90 bar, vorzugsweise 60 bar bis 85 bar. Typische bei der Methanolsynthese verwendete Druckbereiche sind

dem Fachmann hinlänglich bekannt. Für die Erreichung des erforderlichen Synthesedrucks werden der kohlenwasserstoffhaltige Synthesegasstrom und der Kreislaufgasstrom durch eine oder mehrere Kompressorstufen auf den erforderlichen Druck verdichtet.

Rohmethanol enthält Methanol und Wasser sowie gegebenenfalls nicht erwünschte Nebenprodukte. Der Rohmethanolstrom wird insbesondere durch Kühlung, Kondensation und Abscheidung im Sinne einer Gas-Flüssigkeits-Trennung von einem (unter den herrschenden Bedingungen nicht kondensierbaren) Restgasstrom abgetrennt. Der Rohmethanolstrom wird entsprechend insbesondere aus dem Methanolsynthese-Reaktor ausgeschleust und einer weiteren Verarbeitung unterzogen.

Der Methanolsynthese-Reaktor beinhaltet einen geeigneten Methanolsynthese-Katalysator, insbesondere einen festen Katalysator auf Kupfer-Basis. Geeignete Katalysatoren sind dem Fachmann hinlänglich bekannt.

Der Methanolsynthese-Reaktor kann eine oder mehrere Reaktorstufen umfassen. Sind mehrere Reaktorstufen hintereinander angeordnet, so kann nach jeder Reaktorstufe eine Zwischenkondensation des Rohmethanolstroms oder in diesem Fall eines Rohmethanolteilstroms vorgesehen sein.

Durch die Umsetzung im Methanolsynthese-Reaktor wird ein Rohmethanolstrom als Reaktionsprodukt und ein Restgasstrom erhalten. Der Restgasstrom enthält nicht zu Methanol umgesetztes Synthesegas und Kohlenwasserstoffe, da letztere unter den Bedingungen der Methanolsynthese nicht reagieren, unter diesen Bedingungen also inert sind.

Der Restgasstrom wird insbesondere stromabwärts zum Methanolsynthese-Reaktor in einen Kreislaufgasstrom und einen Spülgasstrom aufgeteilt. Der Kreislaufgasstrom wird insbesondere zum Eingang des Methanolsynthese-Reaktors zurückgeführt und vorzugsweise mit dem kohlenwasserstoffhaltigen Synthesegasstrom zusammengeführt, bevor beide Ströme zusammen im Methanolsynthese-Reaktor umgesetzt werden. Der Spülgasstrom ist nicht Teil dieses Kreislaufs, sondern wird insbesondere stromabwärts zum Methanolsynthese-Reaktor aus der Methanolsynthese-Schleife ausgeschleust und gemäß Schritt (f) umgesetzt.

Beim Umsetzten des Spülgasstroms unter Beteiligung von Sauerstoff als Oxidationsmittel in einem Reformierungsschritt zu einem Synthesegasstrom kann vorgehsehen sein, dass dem Spülgasstrom ein Dampfstrom zugeführt wird, so dass der Spülgasstrom und der Dampfstrom im Reformierungsschritt gemäß Schritt (f) zum Synthesegasstrom umgesetzt werden. Die Zuführung von Dampf ermöglicht die Einstellung eines optimalen Kohlenstoff-Dampf-Verhältnisses, beispielsweise für eine partielle Oxidation.

Der elektrolytisch erzeugte Wasserstoffstrom wird insbesondere durch einen Elektrolyseur bereitgestellt. Je nach Verfahrensführung wird ein Teil des elektrolytisch erzeugten Wasserstoffstroms dem kohlenwasserstoffhaltigen Kohlendioxidstrom zugeführt oder der vollständige elektrolytisch erzeugte Wasserstoffstrom dem kohlenwasserstoffhaltigen Synthesegasstrom zugeführt. Vorzugsweise wird der Elektrolyseur so ausgelegt, dass der vollständige elektrolytisch erzeugte Wasserstoffstrom dem kohlenwasserstoffhaltigen Kohlendioxidstrom zugeführt wird. Der Elektrolyseur kann dann die meiste Zeit unter Volllast betrieben werden. Der Elektrolyseur wird dann vorzugsweise so ausgelegt, dass der resultierende kohlenwasserstoffhaltige Synthesegasstrom eine für die Methanolsynthese geeignete Stöchiometriezahl aufweist, beispielsweise eine Stöchiometriezahl SN von 1,9 bis 2,5, vorzugsweise von 2,0 bis 2,4, wobei gilt

$$SN = \frac{n(H_2) - n(CO_2)}{n(CO) + n(CO_2)}, mit\ n\ in\ [mol].$$

Der elektrolytisch erzeugte Wasserstoffstrom kann durch jedes dem Fachmann geläufige Elektrolyse-Verfahren erzeugt werden. Vorzugsweise handelt es sich bei dem Elektrolyse-Verfahren um eine Wasserelektrolyse. Beispiele sind alkalische Elektrolyse, Protonenaustauschermembran-Elektrolyse (PEM-Elektrolyse), Anionenaustauschermembran-Elektrolyse (AEM-Elektrolyse), Hochtemperaturelektrolyse (HTE) und Elektrolyse unter Verwendung von Festoxid-Elektrolyseurzellen (SOEC).

Eine Ausführungsform des erfindungsgemäßen Verfahrens ist dadurch gekennzeichnet, dass der Spülgasstrom vor dem Umsetzen gemäß Schritt (f) einer Wasserstoff-Rückgewinnungseinheit zugeführt wird, wodurch ein mit Kohlenwasserstoffen angereicherter Spülgasstrom sowie ein wasserstoffreicher Strom erhalten werden.

Entsprechend wird gemäß dieser Ausführungsform der mit Kohlenwasserstoffen angereicherte Spülgasstrom gemäß Schritt (f) zu einem Synthesegasstrom umgesetzt.

Die Abtrennung des Wasserstoffs hat zur Folge, dass kein Wasserstoff oder nur minimale Wasserstoffmengen aus dem Spülgasstrom dem Reformierungsschritt gemäß Schritt (f) unterzogen werden. In den Reformierungsschritt gelangender Wasserstoff kann durch die Anwesenheit von Sauerstoff zu Wasser umgesetzt werden, was zu stofflichen Verlusten an Wasserstoff führen kann. Wird der Wasserstoff vorher abgetrennt und anschließend dem kohlenwasserstoffhaltigen Synthesegasstrom wie gemäß Schritt (c) erzeugt zugeführt, werden die Verluste an Wasserstoff diesbezüglich minimiert.

Gemäß einer Ausführungsform wird der wasserstoffreiche Strom daher dem kohlenwasserstoffhaltigen Synthesegasstrom zugeführt.

Eine Ausführungsform des erfindungsgemäßen Verfahrens ist dadurch gekennzeichnet, dass die Wasserstoff-Rückgewinnungseinheit eine Membraneinheit umfasst, wobei der mit Kohlenwasserstoffen angereicherte Spülgasstrom auf der Retentatseite der Membraneinheit erzeugt wird, und der wasserstoffreiche Strom auf der Permeatseite der Membraneinheit erzeugt wird.

Die Verwendung einer Membraneinheit weist den Vorteil auf, dass der mit Kohlenwasserstoffen angereicherte Spülgasstrom ohne signifikante Druckverluste auf der Retentatseite der Membraneinheit erzeugt wird. Dieser Strom kann daher ohne zusätzliche Kompression dem Reformierungsschritt gemäß Schritt (f) zugeführt werden. Wird die Methanolsynthese beispielsweise bei 75 bar betrieben, und wird über die Retentatseite der Membraneinheit samt Rohrleitungen ein Druckverlust von 5 bar angenommen, so weist der mit Kohlenwasserstoffen angereicherte Spülgasstrom einen Druck von circa 70 bar auf. Ein solcher Druck ist für das direkte Einleiten beispielsweise in einen POX-Reaktor geeignet. Dieser Vorteil ist bei Verwendung einer Druckwechseladsorptionsvorrichtung (PSA) nicht realisierbar, da das Desorbieren der "schweren" Komponenten aus dem PSA-Festbett, hier der Kohlenwasserstoffe, stets ein Absenken des Drucks erfordert.

Eine Ausführungsform des erfindungsgemäßen Verfahrens ist dadurch gekennzeichnet, dass der kohlenwasserstoffhaltige Kohlendioxidstrom vor dem Zusammenführen gemäß Schritt (c) zur Entfernung von Schwefelverbindungen in einer Hydrodesulfurierungseinheit behandelt wird.

Da Schwefelverbindungen Katalysatorgifte für Methanolsynthese-Katalysatoren darstellen, kann es je nach Verunreinigungsprofil des kohlenwasserstoffhaltigen Kohlendioxidstroms erforderlich sein, diese Schwefelverbindungen in einer Hydrodesulfurierungseinheit zu entfernen.

Vorzugsweise wird dafür der wasserstoffreiche Strom für die Hydrierung in der Hydrodesulfurierungseinheit genutzt.

Der vom Spülgasstrom durch eine Wasserstoffrückgewinnungseinheit abgetrennte wasserstoffreiche Strom kann auch dem Methanolsynthese-Reaktor zugeführt werden, was jedoch eine Kompression dieses Stroms auf Synthesedruck erfordert. Schließlich fällt dieser wasserstoffreiche Strom, unabhängig davon ob eine Membraneinheit oder eine PSA für dessen Erzeugung genutzt wird, bei niedrigem Druck an.

Da die Hydrierung von Schwefelverbindungen in einer Hydrodesulfurierungseinheit üblicherweise oder vorzugsweise bei niedrigem Druck durchgeführt wird, ist eine zusätzliche Kompression des wasserstoffreichen Stroms in diesem Fall und in vorteilhafter Weise nicht oder zumindest nur in geringem Maße erforderlich.

Wird der wasserstoffreiche Strom dem Methanolsynthese-Reaktor zugeführt, so wird der wasserstoffreiche Strom vorzugsweise dem elektrolytisch erzeugten Wasserstoffstrom vor dem Zusammenführen gemäß Schritt (c) zugeführt wird und/oder dem kohlenwasserstoffhaltigen Synthesegasstrom vor dem Umsetzen gemäß Schritt (d) zugeführt.

Der in beiden Fällen resultierende mit Wasserstoff angereicherte, kohlenwasserstoffhaltige Synthesegasstrom wird vorzugsweise anschließend auf Synthesedruck komprimiert.

Eine Ausführungsform des erfindungsgemäßen Verfahrens ist dadurch gekennzeichnet, dass der Methanolsynthese-Reaktor eine wassergekühlte Reaktorstufe beinhaltet, wobei bei der Kühlung durch die wassergekühlte Reaktorstufe Dampf erzeugt wird, und der Dampf als Prozessdampf für den Reformierungsschritt gemäß Schritt (f) genutzt wird.

Methanolsynthese-Reaktoren können wassergekühlte oder gasgekühlte Reaktorstufen beinhalten. Im Falle von wassergekühlten Reaktorstufen wird häufig siedendes Kesselspeisewasser als Kühlmedium oder anders ausgedrückt Medium zur Temperaturkontrolle der exothermen Methanolbildungsreaktion verwendet. Entsprechend wird am Auslass des Kühlsystems einer solchen Reaktorstufe Dampf, insbesondere Wasserdampf, gebildet. Dieser Dampf kann in vorteilhafter Weise als Prozessdampf für den Reformierungsschritt gemäß Schritt (f) genutzt werden. In einem solchen Fall wird der kohlenwasserstoffhaltige Spülgasstrom unter Beteiligung von Sauerstoff und Dampf zum Synthesegasstrom umgesetzt.

Eine Ausführungsform des erfindungsgemäßen Verfahrens ist dadurch gekennzeichnet, dass ein elektrolytisch erzeugter Sauerstoffstrom bereitgestellt wird, und der Sauerstoff des elektrolytisch erzeugten Sauerstoffstroms als Oxidationsmittel im Reformierungsschritt gemäß Schritt (f) genutzt wird.

Die Verwendung von elektrolytisch erzeugtem Sauerstoff als Oxidatonsmittel hat den Vorteil, dass so erzeugter Sauerstoff eine große Reinheit aufweist. Eine entsprechende Reinheit wird sonst nur durch Gewinnung von Sauerstoff durch kryogene Luftzerlegung erreicht. Jedoch ist ein solcher Luftzerleger apparativ und bezüglich seines Energiebedarfs anspruchsvoll und steht nicht unbedingt zur Verfügung. Ein Wasser-Elektrolyseur bildet hingegen stets Sauerstoff als "Nebenprodukt", welcher normalerweise nicht verwertet wird. Vorzugsweise werden daher der elektrolytisch erzeugte Wasserstoffstrom und der elektrolytisch erzeugte Sauerstoffstrom durch den gleichen Elektrolyseur bereitgestellt. Wird als Oxidationsmittel Luft oder sauerstoffangereicherte Luft verwendet, so hat dies ferner den Nachteil dass durch den anwesenden Stickstoff stets auch unerwünschte Stickoxide im Reformierungsschritt gebildet werden können.

Ein weiterer Vorteil der Verwendung eines elektrolytisch erzeugten Sauerstoffstroms besteht darin, dass in diesem Sauerstoffstrom enthaltene Wasserstoff-Restmengen nicht entfernt werden müssen. Die Anwesenheit von Wasserstoff in einem elektrolytisch erzeugten Sauerstoffstrom ist unvermeidbar auf Grund von Diffusionsprozessen über

die Membranen oder Diaphragmas zwischen den anodischen und kathodischen Bereichen der Elektrolysezellen. Da Wasserstoff und auch nicht vom Sauerstoffstrom abgetrenntes Wasser im Reformierungsschritt gemäß Schritt (f) nicht stören, kann auf eine entsprechende Reinigung des elektrolytisch erzeugten Sauerstoffstroms verzichtet werden.

Eine Ausführungsform des erfindungsgemäßen Verfahrens ist dadurch gekennzeichnet, dass der kohlenwasserstoffhaltige Kohlendioxidstrom durch eine Kohlenstoffabscheideeinheit bereitgestellt wird.

Dadurch wird in der Regel die Reinheit eines primär erzeugten Kohlendioxidstroms in Bezug auf Kohlendioxid stark erhöht. Beispiele von primär erzeugten Kohlendioxidströmen sind Abgasströme oder Rauchgasströme. Ein entsprechender kohlenwasserstoffhaltiger Kohlendioxidstrom eignet sich somit besser für die anschließende Methanolsynthese, da die Bildung von Nebenprodukten vermieden wird. Bei der Kohlenstoffabscheideeinheit *(carbon capture unit)* kann es sich um jede dem Fachmann bekannte Vorrichtung oder Anlage handeln, welche die Bildung eines gereinigten Kohlendioxidstroms ermöglicht. Insbesondere handelt es sich dabei um ein Gaswäscheverfahren, in welchem Kohlendioxid in einem Lösungsmittel mehr oder weniger selektiv entweder physikalisch oder chemisch zunächst absorbiert und anschließend durch Änderung der physikalischen Bedingungen wie Druck und/oder Temperatur oder die Einbringung von Desorptionsmedien wieder desorbiert wird.

Eine Ausführungsform des erfindungsgemäßen Verfahrens ist dadurch gekennzeichnet, dass der gemäß Schritt (d) erzeugte Rohmethanolstrom durch ein thermisches Trennverfahren in Reinmethanol und Wasser getrennt wird, und wobei in dem thermischen Trennverfahren Kohlenwasserstoffe als Nebenproduktstrom abgetrennt werden, und der so erhaltene Nebenproduktstrom dem Spülgasstroms zugeführt wird, zum Umsetzen des Spülgasstroms und des Nebenproduktstroms im Reformierungsschritt gemäß Schritt (f) zu dem Synthesegasstrom.

Das gebildete und aus dem Methanolsynthese-Reaktor ausgeschleuste Rohmethanol wird üblicherweise in einer thermischen Trennvorrichtung, beispielsweise einer Rektifikationskolonne, in Wasser und Methanol getrennt. Da im kohlenwasserstoffhaltigen Synthesegasstrom anwesende Kohlenwasserstoffe unter den Bedingungen der Methanolsynthese inert sind, werden insbesondere mit dem Rohmethanol kondensierte Kohlenwasserstoffe in die thermische Trennvorrichtung verschleppt und müssen dort abgetrennt werden. Auch die an dieser Stelle als Nebenproduktstrom abgetrennte Kohlenwasserstoffe können in vorteilhafter Weise dem Spülgasstrom für die anschließende Umsetzung zu Synthesegas gemäß Schritt (f) zugeführt werden. Die stoffliche Nutzung der Kohlenwasserstoffe wird dadurch weiter verbessert.

Eine Ausführungsform des erfindungsgemäßen Verfahren ist dadurch gekennzeichnet, dass von dem Spülgasstrom ein Spülgasteilstrom abgetrennt wird, und der Spülgasteilstrom aus dem Verfahren ausgeschleust wird.

Dies kann erforderlich sein, wenn auch durch die Umsetzung des Spülgasstroms im Reformierungsschritt gemäß Schritt (f) bestimmte inerte Komponenten im Verfahren, insbesondere in der Methanolsynthese-Schleife, akkumulieren und der durch Schritt (f) erzeugte Synthesegasstrom der Methanolsynthese zugeführt wird. Bei den besagte Verbindungen kann es sich beispielsweise um inerte Verbindungen wie molekularen Stickstoff handeln.

Die vorgenannten Aufgaben werden ferner zumindest teilweise gelöst durch eine Anlage zur Herstellung von Methanol und Synthesegas, umfassend folgende in Wirkverbindung stehende Anlagenkomponenten:

(a) Mittel konfiguriert zum Bereitstellen eines kohlenwasserstoffhaltigen Kohlendioxidstroms;

(b) Ein Elektrolyseur konfiguriert zum Bereitstellen eines elektrolytisch erzeugten Wasserstoffstroms;

(c) Mittel konfiguriert zum Zusammenführen des kohlenwasserstoffhaltigen Kohlendioxidstroms und des elektrolytisch erzeugten Wasserstoffstroms, wodurch ein kohlenwasserstoffhaltiger Synthesegasstrom erhältlich ist;

(d) Ein Methanolsynthese-Reaktor konfiguriert zum Umsetzen des kohlenwasserstoffhaltigen Synthesegasstroms und eines Kreislaufgasstroms, wodurch ein Rohmethanolstrom als Reaktionsprodukt und ein Restgasstrom erhältlich sind, wobei der Restgasstrom nicht zu Methanol umgesetztes Synthesegas und Kohlenwasserstoffe enthält;

(e) Mittel konfiguriert zum Auftrennen des Restgasstroms in den Kreislaufgasstrom und einen Spülgasstrom;

(f) Ein Reaktor konfiguriert zum Umsetzen des Spülgasstroms unter Beteiligung von Sauerstoff als Oxidationsmittel in einem Reformierungsschritt zu einem Synthesegasstrom.

Eine Ausführungsform der erfindungsgemäßen Anlage umfasst eine stromaufwärts zum Reaktor (f) angeordnete Wasserstoff-Rückgewinnungseinheit, und Mittel konfiguriert zum Zuführen des Spülgasstroms zu genannter Wasserstoff-Rückgewinnungseinheit, wobei die Wasserstoff-Rückgewinnungseinheit zur Erzeugung eines mit Kohlenwasserstoffen angereicherten Spülgasstroms und eines wasserstoffreichen Stroms konfiguriert ist, und wobei die Anlage Mittel zum Zuführen des mit Kohlenwasserstoffen angereicherten Spülgasstroms zum Reaktor (f) umfasst.

Eine Ausführungsform der erfindungsgemäßen Anlage ist dadurch gekennzeichnet, dass die Wasserstoff-Rückgewinnungseinheit eine Membraneinheit umfasst, wobei die Membraneinheit so konfiguriert ist, dass der mit Kohlenwasserstoffen angereicherte Spülgasstrom auf der Retentatseite der Membraneinheit erzeugbar ist und der wasserstoffreiche Strom auf der Permeatseite der Membraneinheit erzeugbar ist.

Eine Ausführungsform der erfindungsgemäßen Anlage umfasst weiter eine Hydrodesulfurierungseinheit, wobei die Hydrodesulfurierungseinheit zur Entfernung von Schwefelverbindungen aus dem kohlenwasserstoffhaltigen Kohlendioxidstrom konfiguriert ist.

Eine Ausführungsform der erfindungsgemäßen Anlage ist dadurch gekennzeichnet, dass die Anlage Mittel konfiguriert zur Nutzung des wasserstoffreichen Stroms für die Hydrierung in der Hydrodesulfurierungseinheit umfasst.

Eine Ausführungsform der erfindungsgemäßen Anlage ist dadurch gekennzeichnet, dass die Anlage Mittel zum Zuführen des wasserstoffreichen Stroms zum elektrolytisch erzeugten Wasserstoffstrom aufweist und/oder Mittel konfiguriert zum Zuführen des wasserstoffreichen Stroms zum kohlenwasserstoffhaltigen Synthesegasstrom aufweist.

Eine Ausführungsform der erfindungsgemäßen Anlage ist dadurch gekennzeichnet, dass der Reaktor (f) als POX-Reaktor konfiguriert ist.

Eine Ausführungsform der erfindungsgemäßen Anlage ist dadurch gekennzeichnet, dass der Methanolsynthese-Reaktor eine wassergekühlte Reaktorstufe beinhaltet, wobei bei der Kühlung durch die wassergekühlte Reaktorstufe Dampf erzeugbar ist, und die Anlage Mittel konfiguriert zur Nutzung des Dampfes als Prozessdampf im Reaktor (f) beinhaltet.

Eine Ausführungsform der erfindungsgemäßen Anlage ist dadurch gekennzeichnet, dass der Elektrolyseur zum Bereitstellen eines elektrolytisch erzeugten Sauerstoffstroms konfiguriert ist, und die Anlage Mittel zur Nutzung des elektrolytisch erzeugten Sauerstoffstroms als Oxidationsmittel im Reaktor (f) umfasst.

Eine Ausführungsform der erfindungsgemäßen Anlage ist dadurch gekennzeichnet, dass die Anlage eine Kohlenstoffabscheideeinheit konfiguriert zur Bereitstellung des kohlenwasserstoffhaltigen Kohlendioxidstroms umfasst.

Eine Ausführungsform der erfindungsgemäßen Anlage ist dadurch gekennzeichnet, dass die Anlage eine thermische Trennvorrichtung konfiguriert zur Trennung des Rohmethanolstroms in Reinmethanol und Wasser umfasst, und wobei durch die thermische Trennvorrichtung ein kohlenwasserstoffhaltiger Nebenproduktstrom erzeugbar ist, und die Anlage Mittel konfiguriert zum Zuführen des Nebenproduktstroms zum Spülgasstrom umfasst, wodurch der Spülgasstrom und der Nebenproduktstrom im Reaktor (f) zu dem Synthesegasstrom umsetzbar sind. Eine Ausführungsform der erfindungsgemäßen Anlage ist dadurch gekennzeichnet, dass die Anlage Mittel konfiguriert zum Umsetzen des gemäß (f) erzeugbaren Synthesegasstrom zusätzlich zum Umsetzen des kohlenwasserstoffhaltigen Synthesegasstroms im Methanolsynthese-Reaktor (d) umfasst. Eine Ausführungsform der erfindungsgemäßen Anlage ist dadurch gekennzeichnet, dass die Anlage Mittel zum Abtrennen eines Spülgasteilstroms vom Spülgasstrom umfasst, und die Anlage Mittel zum Ausschleusen des Spülgasteilstroms aus dem Verfahren umfasst.

**Ausführungsbeispiele**

Die Erfindung wird im Folgenden durch Ausführungsbeispiele und Zahlenbeispiele näher erläutert, ohne den Gegenstand der Erfindung dadurch zu beschränken. Weitere Merkmale, Vorteile und Anwendungsmöglichkeiten der Erfindung ergeben sich aus der nachfolgenden Beschreibung der Ausführungsbeispiele in Zusammenhang mit den Figuren und der Zahlenbeispiele. In den Figuren sind funktionell und/oder strukturell gleiche oder zumindest ähnliche Elemente mit gleichen Bezugszeichen versehen.

Es zeigt

Figur 1 ein Blockfließbild eines erfindungsgemäßen Verfahrens zum Herstellen von Methanol und Synthesegas gemäß einem ersten Ausführungsbeispiel,

Figur 2 ein Blockfließbild eines erfindungsgemäßen Verfahrens zum Herstellen von Methanol und Synthesegas gemäß einem zwei-ten Ausführungsbeispiel.

Figur 1 zeigt ein stark vereinfachtes Blockfließbild eines Verfahrens 1 zum Herstellen von Methanol und Synthesegas gemäß einem ersten Ausführungsbeispiel der Erfindung.

Ein kohlenwasserstoffhaltiger und kohlendioxidhaltiger Einsatzgasstrom 20, beispielsweise ein Abgas oder Rauchgas, wird in einer Kohlenstoffabscheideeinheit (*carbon capture unit*) 10 behandelt, um die Kohlendioxidkonzentration im Einsatzgasstrom 20 zu erhöhen. Der resultierende kohlenwasserstoffhaltige Kohlendioxidstrom 21 enthält noch Schwefelverbindungen und wird daher in einer Hydrodesulfurierungseinheit 11 behandelt, um die Schwefelverbindungen quantitativ aus dem Strom 21 zu entfernen. Es resultiert ein schwefelfreier kohlenwasserstoffhaltiger Kohlendioxidstrom 22, welcher mit einem elektrolytisch erzeugten Wasserstoffstrom 24, einem Synthesegasstrom 25 und einem Kreislaufgasstrom 27 zusammengeführt und in einer Kompressionseinheit 12 auf einen für die Herstellung von Methanol geeigneten Synthesedruck verdichtet wird. Der resultierende Strom 23 ist ein kombinierter Strom aus einem

kohlenwasserstoffhaltigen Synthesegasstrom (generiert aus den Strömen 22, 24 und 25) und dem Kreislaufgasstrom 27.

Über Leitung 30 wird ein Rohwasserstrom 30 herangeführt, welcher in einem Wasseraufbereiter 16 behandelt wird, beispielsweise entsalzt wird, woraus ein Reinwasserstrom 29 resultiert, welcher einem Elektrolyseur 15 zugeführt wird. Elektrolyseur 15 ist beispielsweise ein PEM-Elektrolyseur, welcher aus reinem Wasser als Elektrolysemedium einen anodisch erzeugten Sauerstoffstrom 28 und einen kathodisch erzeugten Wasserstoffstrom 24 generiert. Der Wasserstoffstrom 24 wird mit dem kohlenwasserstoffhaltigen (schwefelfreien) Kohlendioxidstrom 22 zusammengeführt, woraus ein kohlenwasserstoffhaltiger Synthesegasstrom resultiert. Dieser kohlenwasserstoffhaltige Synthesegasstrom wird durch den in einem POX-Reaktor 14 erzeugten Synthesegasstrom 25 ergänzt. Der elektrolytisch erzeugte Sauerstoffstrom 28 wird als Oxidationsmittel im POX-Reaktor 14 genutzt. Bei dem Elektrolyseur 15 kann es sich optional um einen Hockdruckelektrolyseur handeln, welcher beispielsweise bei 25 bar betrieben wird. Wir der POX-Reaktor 14 bei entsprechend niedrigem Druck betrieben, beispielsweise 20 bar, kann in vorteilhafter Weise auf die Kompression des Sauerstoffstroms 28 verzichtet werden. Ein Teil des elektrolytisch erzeugten Wasserstoffstroms 24 kann als Teilstrom 24a abgezweigt und der Hydrodesulfurierungseinheit 11 zugeführt werden. Dort wird der elektrolytisch erzeugte Wasserstoffteilstrom 24a für die Hydrierung genutzt.

Der kombinierte Strom 23 enthält Wasserstoff, Kohlendioxid, Kohlenmonoxid und Kohlenwasserstoffe und wird einem Methanolsynthese-Reaktor 13 zugeführt, in welchem die vorgenannten Bestandteile, mit Ausnahme der Kohlenwasserstoffe, an einem geeigneten Methanolsynthese-Katalysator zu Rohmethanol umgesetzt werden. Rohmethanol enthält zumindest Methanol und Wasser. Da die Umsetzung im Methanolsynthese-Reaktor 13 aufgrund der Einstellung eines thermodynamischen Gleichgewichts nicht vollständig ist, wird aus dem Methanolsynthese-Reaktor nicht nur ein Rohmethanolstrom 31, sondern auch ein Restgasstrom 26 ausgeschleust. Der Restgasstrom 26 enthält nicht umgesetzte Synthesegasbestandteile sowie die unter den Bedingungen der Methanolsynthese inerten Kohlenwasserstoffe. Der Restgasstrom 26 wird in einen Spülgasstrom 34 und einen Kreislaufgasstrom 27 aufgeteilt. Der Kreislaufgasstrom 27 ist Teil der Methanolsynthese-Schleife, welche zumindestdurch die Ströme 23, 26 und 27 sowie den Methanolsynthese-Reaktor 13 und die Kompressionseinheit 12 gebildet wird.

Der Spülgasstrom 34 wird aus dieser Schleife, das heißt diesem Kreislaufprozess, ausgeschleust. Er wird nachfolgend dem POX-Reaktor 14 zugeführt, in dem eine Reformierung der Kohlenwasserstoffe des Spülgasstroms mit Hilfe des elektrolytisch erzeugten Sauerstoffstroms 28 und mit einem Dampfstrom (nicht gezeigt) erfolgt. Durch die Reformierung der Kohlenwasserstoffe wird ein Synthesegasstrom 25 erzeugt, welcher Wasserstoff, Kohlenmonoxid und Kohlendioxid enthält. Dieser Synthesegasstrom 25 ergänzt den kohlenwasserstoffhaltigen Synthesegasstrom, welcher durch Kombination der Ströme 22 und 24 erhalten wird. Die im Strom 22 enthaltenen Kohlenwasserstoffe werden so stofflich für die Methanolherstellung im Methanolsynthese-Reaktor 13 genutzt. Bevor der Synthesegasstrom 25 der Kompressionseinheit 12 zugeführt wird, durchläuft dieser eine Kühlstrecke, welche beispielsweise einen Abhitzekessel umfasst (nicht gezeigt). Da durch den Abhitzekessel in der Regel Hochdruckdampf bei ca. 40 bar erzeugt wird, kann dieser Dampf zur Verbesserung der Prozessintegration beispielsweise als Turbinentreibdampf in der Kompressionseinheit 12 oder als Heizmedium in der dem Methanolsynthese-Reaktor nachgeschalteten thermischen Trennvorrichtung 17 verwendet werden.

Der Rohmethanolstrom 31 wird einer thermischen Trennvorrichtung 17, hier einer Rektifikationskolonne 17, zugeführt. In der Rektifikation 17 wird ein Reinmethanolstrom 33 erzeugt, beispielsweise ein Reinmethanolstrom mit einem Gehalt von mindestens 99 Gew.-% Methanol, oder von mindestens 99,5 Gew.-% Methanol. Als weiteres "Produkt" der thermischen Trennung in Rektifikationskolonne 17 fällt Wasser an, welches zur Verbesserung der Prozessintegration beispielsweise als Rohwasserstrom 30 genutzt werden kann (nicht gezeigt). In Rektifikationskolonne 17 fällt auch ein kohlenwasserstoffhaltiger Nebenproduktstrom 32 an, welcher optional zur weiteren Erhöhung der Kohlenstoffausbeute des Verfahrens ebenfalls dem POX-Reaktor 14 zur Umsetzung zu Synthesegas zugeführt werden kann.

Da der Synthesegasstrom 25 der Methanolsynthese-Schleife vollständig als Eduktstrom zugeführt wird, kann es erforderlich sein aus dem Spülgasstrom 34 einen Spülgasteilstrom abzuzweigen und aus dem Verfahren auszuschleusen (nicht gezeigt). Dadurch wird verhindert, dass auch im POX-Reaktor nicht umgesetzte, also inerte Komponenten, in die Methanolsynthese-Schleife gelangen und dort akkumulieren.

Figur 2 zeigt ein stark vereinfachtes Blockfließbild eines Verfahrens 2 zum Herstellen von Methanol und Synthesegas gemäß einem zweiten Ausführungsbeispiel der Erfindung.

Das Verfahren 2 gemäß Figur 2 unterscheidet sich von dem Verfahren 1 gemäß Figur 1 insbesondere durch eine zusätzlich vorhandene Wasserstoffrückgewinnungseinheit 18. Bei der Wasserstoffrückgewinnungseinheit 18 handelt es sich insbesondere um eine Membraneinheit 18. In dieser Membraneinheit 18 wird der kohlenwasserstoffhaltige Spülgasstrom 34 behandelt, bevor er dem POX-Reaktor 14 zugeführt wird. In der Membraneinheit 18 wird permeatseitig ein Wasserstoffstrom 35 erzeugt, und retentatseitig ein entsprechend mit Kohlenwasserstoffen angereicherter (oder *vice versa* an Wasserstoff verarmter) Spülgasstrom 36 erzeugt. Dieser mit Kohlenwasserstoffen angereicherte Spülgasstrom 36 wird dem POX-Reaktor 14 zugeführt. Da Strom 36 frei von Wasserstoff ist oder zumindest an Wasserstoff stark abgereichert ist, wird Wasserstoff entsprechend nicht oder nur in geringem Maße im POX Reaktor mit Sauerstoff zu Wasser umgesetzt, wodurch die Wasserstoffausbeute des Gesamtprozesses weiter verbessert wird. Stattdessen

wird der permeatseitig abgetrennte Wasserstoffstrom 35 der Hydrodesulfurierungseinheit 11 zugeführt. Dies hat insbesondere den Vorteil, dass der Wasserstoffstrom 35 nicht komprimiert werden muss, um als Hydriermittel in der Hydrodesulfurierungseinheit 11 eingesetzt zu werden. Als Alternative könnte der Wasserstoffstrom 35 auch mit in der Kompressionseinheit 12 verdichtet werden und würde dann einen Teil des zu Methanol umgesetzten Synthesegases bilden.

Da der mit Kohlenwasserstoffen angereicherte Spülgasstrom 36 retentatseitig in der Membraneinheit 18 erzeugt wird, weist dieser keinen signifikanten Druckverlust gegenüber dem Druck im Methanolsynthese-Reaktor 13 auf. Strom 36 muss daher nicht eigens verdichtet werden, bevor dieser in den POX-Reaktor 14 eingeschleust werden kann.

Die folgenden Zahlenbeispiele basieren auf Simulationsdaten und dienen der weiteren Erläuterung der Erfindung. Die Simulationsdaten wurden mit Hilfe der Software AspenPlus® generiert.

Die folgende Tabelle zeigt zunächst ein Beispiel für eine typische Konzentrationen an Kohlenwasserstoffen in dem kohlenwasserstoffhaltigen Kohlendioxidstrom 22 und wie sich diese im Verfahren verändert. Im Beispiel wird ein Kohlendioxidstrom mit einem Anteil von 5 Vol.-% Methan angenommen.

| | **Strom 22** | **Strom 22+24** | **Strom 27** | **Strom 34** |
|---|---|---|---|---|
| **Anteil Methan / Vol.-%** | 5,00 | 1,30 | 17,2 | 17,2 |
| **Normalisierter Volumenstrom / %** | 100 | 386 | 1737 | 26 |
| **Druck / bara** | 1,0 | 80,0 | 75,0 | 75,0 |

Die ursprüngliche Konzentration von 5 Vol.-% Methan im kohlenwasserstoffhaltigen Kohlendioxidstrom 22 verringert sich durch die Zugabe des elektrolytisch erzeugten Wasserstoffstroms 25 auf 1,30 Vol.-%. Jedoch reichert sich das Methan durch die Umsetzung zu Methanol in der Methanolsynthese-Schleife stark an, was an den Konzentrationen von 17,2 Vol.-% für den Kreislaufgasstrom 27 und den Spülgasstrom 34 erkennbar ist. Dieser Anteil ist bedeutend höher als in Strom 22 oder im kombinierten Strom 22+24. Durch den geringen Volumenstrom des Spülgasstroms 34 eignet sich dieser als Einsatzgasstrom für den POX-Reaktor, da dieser dadurch klein und somit besonders effizient und darüber hinaus kostengünstig ausgelegt werden kann.

Für die folgenden Vergleichsbeispiele wird angenommen, dass Kohlenwasserstoffe aus dem Spülgasstrom 34 und Kohlenwasserstoffe des Nebenproduktstroms 32 vollständig verbrannt würden, anstatt diese gemäß der Erfindung zu verwerten. Auf dieser Basis werden die normalisierten Kohlendioxidemissionen für die Vergleichsbeispiele angegeben und auf 100 % gesetzt.

Die folgende Tabelle zeigt den Vergleich eines Vergleichsbeispiels 1 mit einem erfindungsgemäßen Beispiel 1 für die Integration eines POX-Reaktors für den Spülgasstrom 34 bei 5 Vol.-% Methan im Strom 22.

| | **Vergleichsbeispiel 1** | **Beispiel 1** |
|---|---|---|
| Normalisierte Methanol-Kapazität der Anlage | 100 % | 105 % |
| Normalisierte Kohlendioxid-Emission | 100 % | 23 % |

Durch die Nutzung der Kohlenwasserstoffe des Spülgasstroms zur Erzeugung von Synthesegas durch den POX-Reaktor und anschließender Nutzung dieses Synthesegases in der Methanolsynthese werden somit 77 % der Kohlendioxidemissionen eingespart. Darüber hinaus wird die Methanolkapazität der Anlage durch die stoffliche Nutzung des gebundenen Kohlenstoffs und Wasserstoffs um 5 % erhöht.

Die folgende Tabelle zeigt den gleichen Fall für einen Anteil von 10 Vol.-% Methan im Strom 22.

| | **Vergleichsbeispiel 2** | **Beispiel 2** |
|---|---|---|
| Normalisierte Methanol-Kapazität der Anlage | 100 % | 117 % |
| Normalisierte Kohlendioxid-Emission | 100 % | 22 % |

Die folgende Tabelle zeigt den gleichen Fall für einen Anteil von 2,5 Vol.-% Methan und 2,5 Vol.-% weiteren Kohlenwasserstoffen im Strom 22. Die weiteren Kohlenwasserstoffe setzen sich dabei wie folgt zusammen: 1 Vol.-% Ethan, 0,75 Vol.-% Propan, 0,5 Vol.-% Butan und 0,25 Vol.-% Pentan.

| | Vergleichsbeispiel 3 | Beispiel 3 |
|---|---|---|
| Normalisierte Methanol-Kapazität der Anlage | 100 % | 105 % |
| Normalisierte Kohlendioxid-Emission | 100 % | 25 % |

Die folgende Tabelle zeigt den Vorteil gegenüber Beispiel 3, wenn der Spülgasstrom 34 zusätzlich in einer Membraneinheit 18 behandelt wird und der retentatseitig erhaltene, mit Kohlenwasserstoffen angereicherte Spülgasstrom 36 dem POX-Reaktor 14 zugeführt wird.

| | Vergleichsbeispiel 4 | Beispiel 4 |
|---|---|---|
| Normalisierte Methanol-Kapazität der Anlage | 100 % | 109 % |
| Normalisierte Kohlendioxid-Emission | 100 % | 21 % |

Überraschenderweise wird bezüglich Beispiel 4 festgestellt, abgesehen von den bereits erwähnten Vorteilen, dass durch die zusätzliche Membraneinheit 18 die Methanolkapazität gegenüber Beispiel 3 nochmals gesteigert werden kann und die Emissionen gesenkt werden können.

**Bezugszeichenliste**

| | |
|---|---|
| 1, 2 | Verfahren |
| 10 | Kohlenstoffabscheideeinheit |
| 11 | Hydrodesulfurierungseinheit |
| 12 | Kompressionseinheit |
| 13 | Methanolsynthese-Reaktor |
| 14 | POX-Reaktor |
| 15 | Elektrolyseur |
| 16 | Wasseraufbereiter |
| 17 | Rektifikation |
| 18 | Wasserstoffrückgewinnungseinheit (Membraneinheit) |
| 20 | Einsatzgasstrom |
| 21 | Kohlenwasserstoffhaltiger Kohlendioxidstrom (schwefelhaltig) |
| 22 | Kohlenwasserstoffhaltiger Kohlendioxidstrom (schwefelfrei) |
| 23 | Kombinierter Strom aus kohlenwasserstoffhaltigem Synthesegasstrom und Kreislaufgasstrom |
| 24, 24a | Elektrolytisch erzeugter Wasserstoffstrom |
| 25 | Synthesegasstrom |
| 26 | Restgasstrom |
| 27 | Kreislaufgasstrom |
| 28 | Elektrolytisch erzeugter Sauerstoffstrom |
| 29 | Reinstwasserstrom |
| 30 | Rohwasserstrom |
| 31 | Rohmethanolstrom |
| 32 | Nebenproduktstrom |
| 33 | Reinmethanolstrom |
| 34 | Spülgasstrom |
| 35 | Wasserstoffstrom |
| 36 | Mit Kohlenwasserstoffen angereicherter Spülgasstrom |

**Patentansprüche**

**1.** Verfahren (1, 2) zum Herstellen von Methanol und Synthesegas, umfassend die folgenden Verfahrensschritte:

(a) Bereitstellen eines kohlenwasserstoffhaltigen Kohlendioxidstroms (22);

(b) Bereitstellen eines elektrolytisch erzeugten Wasserstoffstroms (24);
(c) Zusammenführen der Ströme aus Schritt (a) und (b) zu einem kohlenwasserstoffhaltigen Synthesegasstrom;
(d) Umsetzen des kohlenwasserstoffhaltigen Synthesegasstroms und eines Kreislaufgasstroms (27) in einem Methanolsynthese-Reaktor (13), wobei ein Rohmethanolstrom (31) als Reaktionsprodukt und ein Restgasstrom (26) erhalten werden, wobei der Restgasstrom nicht zu Methanol umgesetztes Synthesegas und Kohlenwasserstoffe enthält;
(e) Auftrennen des Restgasstroms in den Kreislaufgasstrom (27) und einen Spülgasstrom (34);
(f) Umsetzen des Spülgasstroms (34) unter Beteiligung von Sauerstoff als Oxidationsmittel in einem Reformierungsschritt zu einem Synthesegasstrom (25).

2. Verfahren nach Anspruch 1, **dadurch gekennzeichnet, dass** der Spülgasstrom (34) vor dem Umsetzen gemäß Schritt (f) einer Wasserstoff-Rückgewinnungseinheit (18) zugeführt wird, wodurch ein mit Kohlenwasserstoffen angereicherter Spülgasstrom (36) sowie ein wasserstoffreicher Strom (35) erhalten werden.

3. Verfahren nach Anspruch 2, **dadurch gekennzeichnet, dass** die Wasserstoff-Rückgewinnungseinheit (18) eine Membraneinheit umfasst, wobei der mit Kohlenwasserstoffen angereicherte Spülgasstrom (36) auf der Retentatseite der Membraneinheit erzeugt wird, und der wasserstoffreiche Strom (35) auf der Permeatseite der Membraneinheit erzeugt wird.

4. Verfahren nach einem der vorhergehenden Ansprüche, **dadurch gekennzeichnet, dass** der kohlenwasserstoffhaltige Kohlendioxidstrom (22) vor dem Zusammenführen gemäß Schritt (c) zur Entfernung von Schwefelverbindungen in einer Hydrodesulfurierungseinheit (11) behandelt wird.

5. Verfahren nach einem der Ansprüche 2 bis 4, **dadurch gekennzeichnet, dass** der wasserstoffreiche Strom (35) für die Hydrierung in der Hydrodesulfurierungseinheit genutzt wird.

6. Verfahren nach einem der Ansprüche 2 bis 4, **dadurch gekennzeichnet, dass** der wasserstoffreiche Strom (35) dem elektrolytisch erzeugten Wasserstoffstrom vor dem Zusammenführen gemäß Schritt (c) zugeführt wird und/oder dem kohlenwasserstoffhaltigen Synthesegasstrom vor dem Umsetzen gemäß Schritt (d) zugeführt wird.

7. Verfahren nach einem der vorhergehenden Ansprüche, **dadurch gekennzeichnet, dass** der Reformierungsschritt eine partielle Oxidation beinhaltet.

8. Verfahren nach einem der vorhergehenden Ansprüche, **dadurch gekennzeichnet, dass** der Methanolsynthese-Reaktor (13) eine wassergekühlte Reaktorstufe beinhaltet, wobei bei der Kühlung durch die wassergekühlte Reaktorstufe Dampf erzeugt wird, und der Dampf als Prozessdampf für den Reformierungsschritt gemäß Schritt (f) genutzt wird.

9. Verfahren nach einem der vorhergehenden Ansprüche, **dadurch gekennzeichnet, dass** ein elektrolytisch erzeugter Sauerstoffstrom (28) bereitgestellt wird, und der Sauerstoff des elektrolytisch erzeugten Sauerstoffstroms (28) als Oxidationsmittel im Reformierungsschritt gemäß Schritt (f) genutzt wird.

10. Verfahren nach einem der vorhergehenden Ansprüche, **dadurch gekennzeichnet, dass** der kohlenwasserstoffhaltige Kohlendioxidstrom (22) durch eine Kohlenstoffabscheideeinheit (10) bereitgestellt wird.

11. Verfahren nach einem der vorhergehenden Ansprüche, **dadurch gekennzeichnet, dass** der gemäß Schritt (d) erzeugte Rohmethanolstrom (31) durch ein thermisches Trennverfahren in Reinmethanol (33) und Wasser getrennt wird, und wobei in dem thermischen Trennverfahren Kohlenwasserstoffe als Nebenproduktstrom (32) abgetrennt werden, und der so erhaltene Nebenproduktstrom (32) dem Spülgasstroms (34) zugeführt wird, zum Umsetzen des Spülgasstroms und des Nebenproduktstroms im Reformierungsschritt gemäß Schritt (f) zu dem Synthesegasstrom (25).

12. Verfahren nach einem der vorhergehenden Ansprüche, **dadurch gekennzeichnet, dass** der gemäß Schritt (f) erzeugte Synthesegasstrom (25) zusätzlich zum kohlenwasserstoffhaltigen Synthesegasstrom (22) im Methanolsynthese-Reaktor umgesetzt wird.

13. Anlage zur Herstellung von Methanol und Synthesegas, umfassend folgende in Wirkverbindung stehende Anlagenkomponenten:

(a) Mittel konfiguriert zum Bereitstellen eines kohlenwasserstoffhaltigen Kohlendioxidstroms (22);
(b) Ein Elektrolyseur (15) konfiguriert zum Bereitstellen eines elektrolytisch erzeugten Wasserstoffstroms (24);
(c) Mittel konfiguriert zum Zusammenführen des kohlenwasserstoffhaltigen Kohlendioxidstroms (22) und des elektrolytisch erzeugten Wasserstoffstroms (24), wodurch ein kohlenwasserstoffhaltiger Synthesegasstrom erhältlich ist;
(d) Ein Methanolsynthese-Reaktor (13) konfiguriert zum Umsetzen des kohlenwasserstoffhaltigen Synthesegasstroms und eines Kreislaufgasstroms (27), wodurch ein Rohmethanolstrom (31) als Reaktionsprodukt und ein Restgasstrom (26) erhältlich sind, wobei der Restgasstrom nicht zu Methanol umgesetztes Synthesegas und Kohlenwasserstoffe enthält;
(e) Mittel konfiguriert zum Auftrennen des Restgasstroms (26) in den Kreislaufgasstrom (27) und einen Spülgasstrom (34);
(f) Ein Reaktor (14) konfiguriert zum Umsetzen des Spülgasstroms (34) unter Beteiligung von Sauerstoff als Oxidationsmittel in einem Reformierungsschritt zu einem Synthesegasstrom (25).

**14.** Anlage nach Anspruch 13, weiter umfassend eine stromaufwärts zum Reaktor (f) (14) angeordnete Wasserstoff-Rückgewinnungseinheit (18), und Mittel konfiguriert zum Zuführen des Spülgasstroms (34) zu genannter Wasserstoff-Rückgewinnungseinheit (18), wobei die Wasserstoff-Rückgewinnungseinheit (18) zur Erzeugung eines mit Kohlenwasserstoffen angereicherten Spülgasstroms (36) und eines wasserstoffreichen Stroms (35) konfiguriert ist, und wobei die Anlage Mittel zum Zuführen des mit Kohlenwasserstoffen angereicherten Spülgasstroms (36) zum Reaktor (f) (14) umfasst.

**15.** Anlage nach Anspruch 14, wobei die Wasserstoff-Rückgewinnungseinheit (18) eine Membraneinheit umfasst, wobei die Membraneinheit so konfiguriert ist, dass der mit Kohlenwasserstoffen angereicherte Spülgasstrom (36) auf der Retentatseite der Membraneinheit erzeugbar ist und der wasserstoffreiche Strom (35) auf der Permeatseite der Membraneinheit erzeugbar ist.

**16.** Anlage nach einem der Ansprüche 13 bis 15, wobei der Reaktor (f) (14) als POX-Reaktor konfiguriert ist.

**17.** Anlage nach einem der Ansprüche 13 bis 16, wobei die Anlage Mittel konfiguriert zum Umsetzen des gemäß (f) erzeugbaren Synthesegasstrom (25) zusätzlich zum Umsetzen des kohlenwasserstoffhaltigen Synthesegasstroms im Methanolsynthese-Reaktor (d) (13) umfasst.

Fig. 1

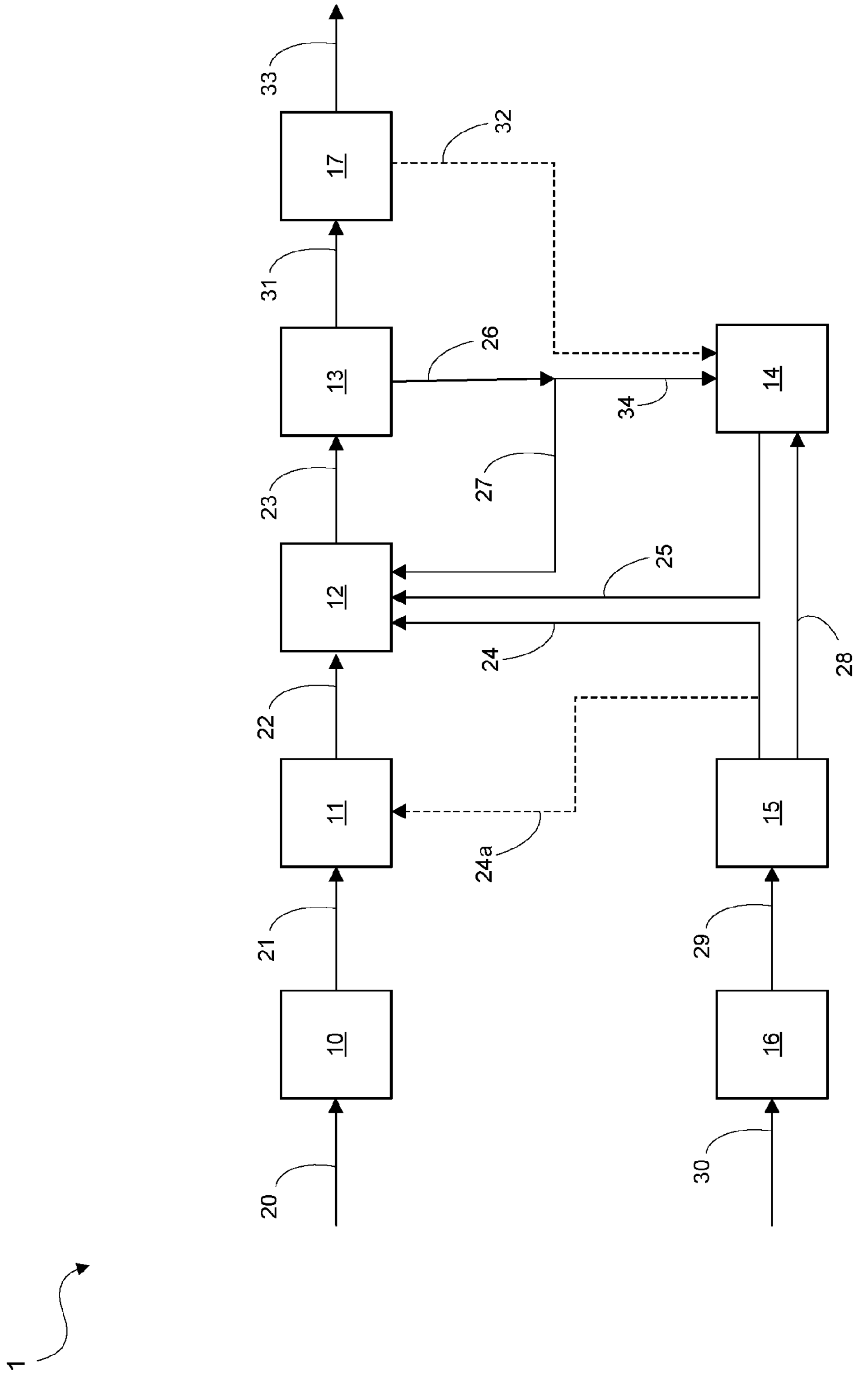
1
10
11
12
13
14
15
16
17
20
21
22
23
24
24a
25
26
27
28
29
30
31
32
33
34

Fig. 2

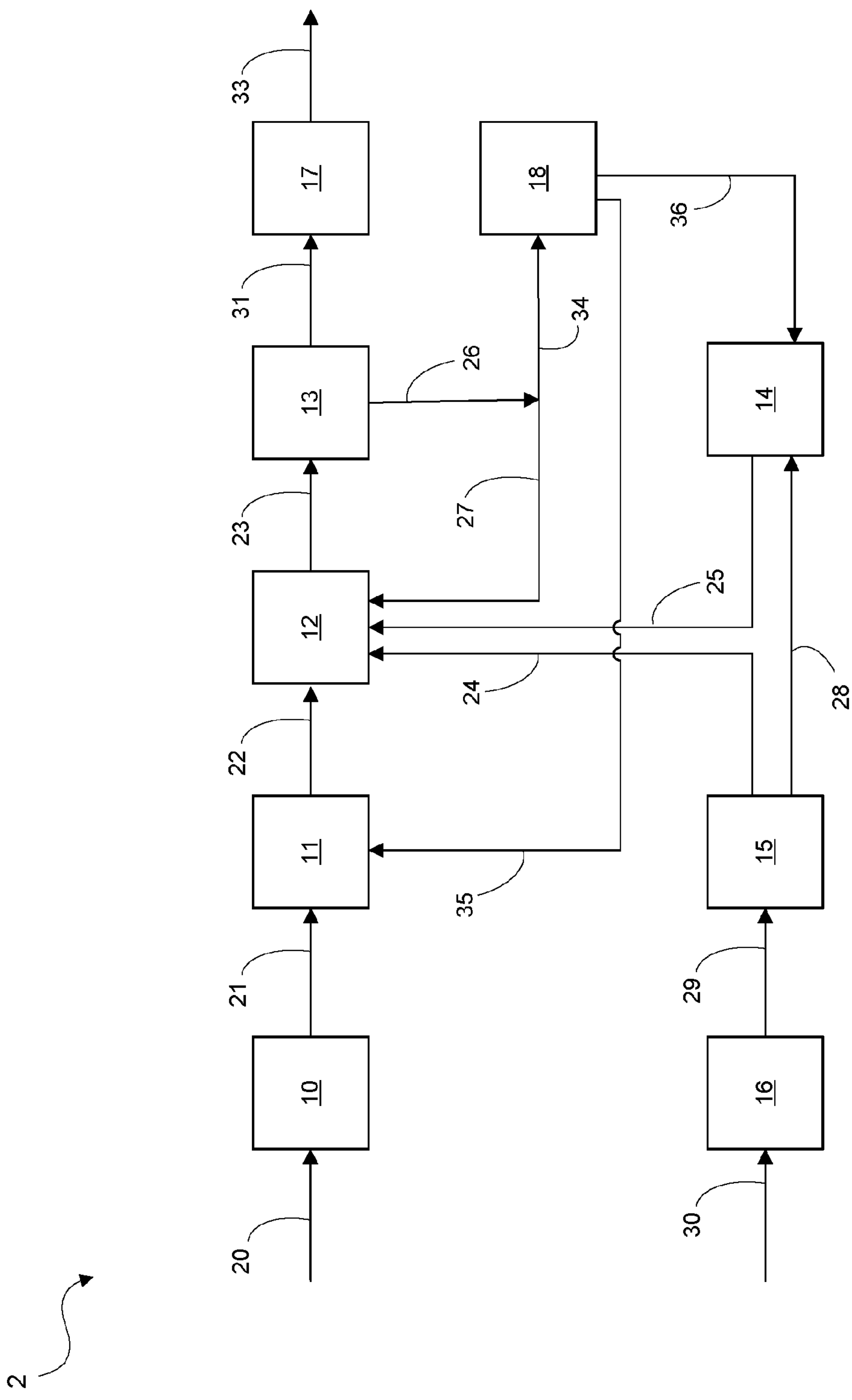
10
11
12
13
14
15
16
17
18
20
21
22
23
24
25
26
27
28
29
30
31
33
34
35
36
2

Europäisches Patentamt
European Patent Office
Office européen des brevets

# EUROPÄISCHER RECHERCHENBERICHT

Nummer der Anmeldung
EP 22 19 0843

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| Y | WO 2020/229261 A1 (THYSSENKRUPP IND SOLUTIONS AG [DE]; THYSSENKRUPP AG [DE]) 19. November 2020 (2020-11-19)<br>* Ansprüche; Abbildung 4 *<br>* Seite 13, letzter Absatz *<br>* Seite 15, Absatz 2 *<br>----- | 1-17 | INV.<br>C07C29/151<br>C07C31/04<br>C25B1/04<br>C01B3/36 |
| Y | WO 2022/079010 A1 (HALDOR TOPSOE AS [DK]) 21. April 2022 (2022-04-21)<br>* Ansprüche *<br>* Seite 3, Zeile 22 *<br>* Seite 4, Zeilen 13-15 *<br>* Seite 17, Zeilen 23-35 *<br>* Seite 20, Zeilen 10-14 *<br>----- | 1-17 | |
| X | WO 2020/048809 A1 (BASF SE [DE]) 12. März 2020 (2020-03-12)<br>* Seiten 24-26; Abbildung 4 *<br>* Seite 9, Absatz 1 *<br>* Seite 26, Zeilen 35-38 *<br>* Beispiele; Tabellen *<br>----- | 1,2,6, 10,11, 13,14 | |

| RECHERCHIERTE SACHGEBIETE (IPC) |
|---|
| C07C<br>C25B<br>C01B |

Der vorliegende Recherchenbericht wurde für alle Patentansprüche erstellt

2

| Recherchenort | Abschlußdatum der Recherche | Prüfer |
|---|---|---|
| Den Haag | 30. Januar 2023 | Kardinal, Siegmar |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

## ANHANG ZUM EUROPÄISCHEN RECHERCHENBERICHT ÜBER DIE EUROPÄISCHE PATENTANMELDUNG NR.

EP 22 19 0843

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

30-01-2023

| Im Recherchenbericht angeführtes Patentdokument | Datum der Veröffentlichung | Mitglied(er) der Patentfamilie | Datum der Veröffentlichung |
|---|---|---|---|
| WO 2020229261 A1 | 19-11-2020 | AU 2020274854 A1 | 16-12-2021 |
| | | CA 3140518 A1 | 19-11-2020 |
| | | CN 113840818 A | 24-12-2021 |
| | | DE 102019113003 A1 | 19-11-2020 |
| | | EP 3969433 A1 | 23-03-2022 |
| | | JP 2022533602 A | 25-07-2022 |
| | | US 2022162143 A1 | 26-05-2022 |
| | | WO 2020229261 A1 | 19-11-2020 |
| WO 2022079010 A1 | 21-04-2022 | KEINE | |
| WO 2020048809 A1 | 12-03-2020 | CN 112638849 A | 09-04-2021 |
| | | EP 3847146 A1 | 14-07-2021 |
| | | US 2021363007 A1 | 25-11-2021 |
| | | WO 2020048809 A1 | 12-03-2020 |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 2016034344 A1